# EUROPEAN PATENT APPLICATION

(11) **EP 2 273 399 A2**
(43) Date of publication of application: **12.01.2011**
(21) Application number: 10167084.2
(22) Date of filing: 23.06.2010
(51) Int. Cl.: G06F 19/00

(54) **Electronic backbone for medicine infrastructure**

(30) Priority: 23.06.2009 US 219752 P; 23.07.2009 US 510173
(71) Applicant: Sony Corporation, Minato-ku Tokyo 108-0075 (JP); Sony DADC US Inc., Terra Haute, IN 47804 (US)
(72) Inventor: Selinfreund, Richard, Terre Haute, IN 47803 (US); Macdonald, John S., Terre Haute, IN 47803 (US); New, Anthony C., Terre Haute, IN 47805 (US)
(74) Representative: McGowan, Cathrine

(57) **Abstract**

Embodiments of the invention relate to the association of a non-guessable, unique ID with a user and the automatic or electronic referral of user ID to a medical service provider, without revealing private medical information. After the assignment of a unique ID to a User, the ID may then provide authentication by a certified health care source. In some embodiments, the delivery of a unique ID to a User can act as a token that can provide authentication for data entering a secure transmissions system for medical referrals and tracking. In some embodiments, an electronic system compensates various parties by tracking activities associated with a unique user ID number without revealing any private medical information of a user also associated with the ID.

## Description

### Background

This invention is generally related to the coordination of medical care. At present, there are no scalable systems which allow the confidential transfer and tracking of medical infrastructure commands that does not transmit electronic medical records (EMRs).

Whether determining if tens of billions of dollars in relief aid have been effectively utilized, or if a single patient is progressing in a preventative care program, there is a critical need for a confidential, effective and scalable system for the distribution and transfer of medical management commands..

### Summary

Aspects and embodiments of the invention are set out in the appended claims. As further detailed herein, some embodiments of the present invention relate to the association of a non-guessable, unique ID with a user and the automatic or electronic referral of user ID to a medical service provider without revealing private medical information. In some embodiments, after the assignment of a unique ID to a User, the ID may then provide authentication by a certified health care source. In some embodiments, once a unique ID is assigned to a User, the ID can act as a token to provide confidential up to date data regarding a specific need either as a fixed element such as a CD-ROM, DVD, BD or as a disc/token to allow streaming of up-to-date information over the Internet or other electronic interface. In some embodiments, the delivery of a unique ID to the User can act as a token that can be an authentication token entering a secure e-Backbone for medical referrals and tracking. In some embodiments, data regarding treatment and/or follow-up is transmitted or communicated without user specific medical information. In some embodiments, an electronic infrastructure is contemplated which can electronically deliver and manage medically related data without storing any user specific medical information. In some embodiments, an electronic system is contemplated by which compensation can be provided to a first entity, service provider through the e-Backbone. In some embodiments, an electronic system compensates various parties by tracking activities associated with a unique user ID number without revealing any private medical information of a user also associated with the ID.

### Brief Description of the Drawings

Embodiments of the invention will now be described with reference to the accompanying drawings, throughout which like parts are referred to by like references, and in which:
Figure 1 illustrates basic exemplary steps and objects/entities that may be used for different functions and embodiments.
Figure 2 illustrates additional basic exemplary steps and objects/entities.
Figure 3 shows a flowchart illustrating basic exemplary steps in a particular embodiment.

### Detailed Description of Embodiments

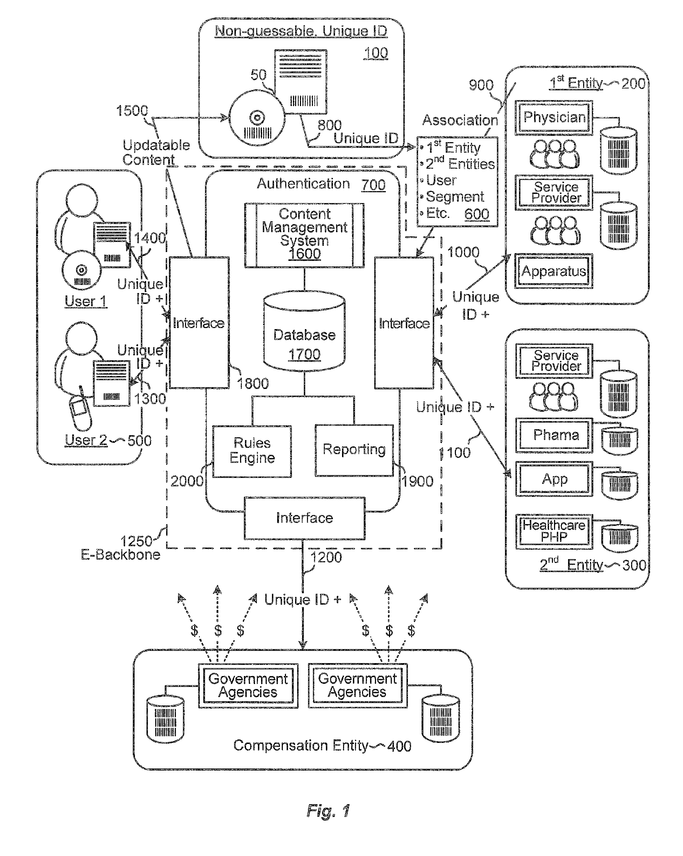

As detailed below, Figure 1 details the manufacture and distribution of non guessable ID to medical touch points at various points in the health care chain.

In some embodiments, a first entity 200 associates a non guessable ID or number 800 to user 900. The non-guessable ID is associated with a user at first entity 200. The first entity 200 could be comprised of one or more of physicians, service providers, or an apparatus. In the event that the first entity is an apparatus, a transfer could be initiated by measuring user data to a reference standard. First entity could be a system of measuring physiological data from time initial to monitor that the measurement does not significantly change from initial or from an a reference standard. In one example a first entity could be a person or apparatus that generates Hemoglobin A1c or blood cell type count measurements made at a clinic, home, any care setting taken by a health care giver or even the user. In a second example a first entity could be a remote or direct measuring device that measures physiological data like EKG, blood cell type count, protein maker level. In this case, the blood cell type count of a discrete sample could be compared to a reference standard sample. As markers or key predictors for additional conditions are found, similar systems would be employed. These conditions include but are not limited to ageing; bone health and osteoporosis; cancer; diabetes; digestive health; external appearance; immune cell depression disorders, fatigue; fertility; stress: performance, heart and cardiovascular health.

The association of the non guessable ID can happen at any point in the E-Backbone as long as numbers are non duplicated or that assignment of multiple IDs to a single user for a single health initiative is not repeated, e.g. at 600 by combining 800 with 900. In the event that a user loses or needs a new disc, the first or second entity may assign a user one ore more additional ID's. In some embodiments, the e-Backbone 1250 will not know the user by name, however, will be able to recognize parent-child relationships between ID's, where the activation of one ID may lead to the de-activation of another, or allow for multiple ID's to remain active for the same user.

A non-guessable ID or number may be defined to be serial numbers that are assigned to a specific group or purpose and are typically simple values that monotonically increase from a start value. When information that is shared across a network or between different applications has been assigned a serialized ID, the need to protect the structure/format of and/or authenticate the serialized ID being passed, is required. For example, knowing the specific value of a serialized ID assigned to a data set containing information associated with a specific purpose or entity should not allow the understanding of any other serialized ID assigned to a different data set. In order to hide the structure/format of the serialized ID, methods can be utilized that make the serial ID non-guessable. A non-guessable identifier is one in which the probability of guessing the value of other serialized IDs is very low.

In some cases, encryptions schemes (AES, Twofish, Serpent, etc) are used to scramble simple monotonically increasing values such that the values appear random. By knowing the encryption key, the original serialized ID can be decrypted, authenticated and used for its original intent by the authentication side. For example, a set of monotonically increasing 128 bit values can be randomized by applying AES encryption to the values. The encryption algorithm in concert with the predefined 128 bit encryption key (can also use a 192 bit or 256 bit keys) modifies the 128 bit values (plain-text) so that they appear random (cipher-text). From Wikipedia (http://en.wikipedia.org/wiki/Advanced Encryption Standard): "The AES cipher is specified as a number of repetitions of transformation rounds that convert the input plain-text into the final output of cipher-text. Each round consists of several processing steps, including one that depends on the encryption key. A set of reverse rounds are applied to transform cipher-text back into the original plain-text using the same encryption key."

In other cases, pseudo-random number generation is used to make serialized ID values not predictable/guessable. In this case, the list of serialized IDs generated must be stored by the authentication side of the message. When the serialized ID is passed the authentication side compares the pseudo-randomly generated value to the predefined list of values and makes a determination if the value is authentic. If authentic, then the expected behavior can occur. If not authenticated, then the expected behavior is not allowed.

In simple terms, the difference between guessable and non-guessable values is that a set of guessable values will have a repeating pattern that can be recognized and understood. A set of non-guessable values will not have a noticeable pattern and will appear random.

When tied to the non-guessable ID, the user may be given a user ID, disc, or any device that provides information or allows access to information with medical relevance as shown in figure 1, 1300 and 1400. The token may later be used to authenticate the user at other locations and provide authentication when connected to the E-Backbone at any point for example 300.

The token, disc, CD-ROM, DVD, BD could be used as an authentication device to send updated information to the user or updated content when they are connected to the e-Backbone 1250 through the digital element 1500. This enables a health care provider to provide relevant information to a patient, based on the patient's status within a given treatment program.

This is especially important when patients live in remote areas or in areas where there is great cost associated with visiting a medical professional. In some embodiments, as contemplated herein, medical professionals, especially in underdeveloped communities receiving foreign medical aid, are able to track a patient's progress, automatically updating individual medical records on their host computer, without requiring a physical visit to their office. In these environments, where medical professionals could be responsible for the health of thousands of patients each, this system provides a scalable way of tracking and managing each patient's health. It allows patients with the greatest needs to be prioritized for a visit to the medical office. Further, each ID may be associated to recommendations per specific status changes. The system may run reports for the first entity tracking progress through specific treatment programs, especially for preventative disease programs, allowing real time, dynamic, medical referrals and recommendations. In some embodiments, patients in remote locations visit a local station having a computing device capable of communication with the e-Backbone 1250. In those situations, patient progress data is stored locally and the e-Backbone 1250 facilitates transmissions with the medical professionals locally stored electronic records. In some embodiments, where there is no local storage available, data is input telephonically to either a third party remote data server, or directly to the medical providers database.

In some embodiments, the system includes an authentication server that uses an electronic infrastructure 700. The authentication server may have a content management system 1600 that is certified by a first entity 200 and/or service providers 300 and/or a compensation entity 400. The authentication database 1700 has a critical role in associating the user ID with non guessable unique ID. The electronic Interface elements 1800 allow the unique IDs to move bi directionally from the database to the first entity 1000, to the second entities, e.g. service providers 1100, to the user 1300 and to compensation entity 1200. The rules engine provides rules, and, if called for, commands for validation of providers, authentication, certification, compensation. The Reporting element 1900 provides an electronic schedule to be used for compensation to second entity 300 and first entity 200 based on authorization of information from the business rules engine regarding non user specific medical information.

The association event at the first entity could initiate a referral to a second entity. The association event could happen at any place along the e-Backbone 1250 where the user is associated with a non guessable ID. No transfer of user specific personal and/or medical information is implied in this diagram. In some embodiments, data may be sent to an authorized electronic storage facility, e.g. Iron Mountain^{™} (www.ironmountain.com), and the e-Backbone 1250 may coordinate new entries with the first and second entities. The associated ID information may enter the e-Backbone 1250 a number of ways. Primarily, information will be transferred electronically with information described in 600, through the authentication server using a web interface. Information may also enter the system through a telephonic or audio only interface. Information may also be physically provided non-electronically e.g. walking information from one party to another, mailing physical data from one party to another, etc.. After the initial association steps 800 and 900 the token can be provided to the user to travel using physical methods to other elements of the digital backbone that are interconnected. It is not implied that all communication travel through the E-Backbone. Registration could be automatic in health programs without human decisions being required as in a fist entity 200 that is only an apparatus.

Second entities 300 could be one or more service providers, pharmacies, apparatus', healthcare providers, non profit organizations, recognized heath care program in a community, etc. A second entity, broadly defined, is any receiver of a referred authentication data transmission from an authenticated source. A second entity can take an action based on the data source origin or code number without reference to specific physiological data or the person from whom the data was obtained. A second entity may be the directed target of a digital transmission of non user changeable code that can be used as an authentication indicia from the first entity. Referrals can travel to the link 1100 and the information can provide authentication of the users and allow for compensation through link 1200. Second entities can allow users to interact with other users during a benefit program or after benefit services by the rules defined by the rules engine 2000.

Compensation entity 400 provides e-Backbone 1250 information for compensation using the rules employed by the specific government agency, non-governmental organizations, insurance companies, or any other compensation entity. The compensation action could, but is not required to, provide information to the Interface 1200. It is noted that this system could be used to convert a compensation system based on success rather than a compensation system based on services.

With further respect to compensation, commercial health plans (Blue Cross and Blue Shield, Aetna, etc.) and government health programs and/or non governmental health care providers (NGOs) (Medicare and Medicaid and TRICARE, Governmental and NGOs pay for health care in two ways; as covered benefits and as administrative efforts. A covered benefit is a service that is provided by a health care provider to a beneficiary that is described as a covered benefit in the benefit certificate. In these instances, health plans compensate the provider through reimbursement (i.e. payment for covered services rendered to the covered beneficiary). Most typically, one thinks of reimbursement to a doctor or hospital or laboratory for a service provided. However, there are several examples of digital communication of information being incorporated into the global reimbursement for a service, such as when an x-ray is transmitted via a T1 line, that digital manipulation is contained in the global payment for the reading of the x-ray. There are examples of digital communication being the entire service, as well as in the case of cardiac pacemaker. Pacemakers require regular checking of pacemaker programming as well as battery status. Pacemaker patients have regular (i.e. approximately monthly) telephonic evaluations of their pacemaker when the phone is put in a cradle device and a transponder that is attached to the cradle communicates with the pacemaker, determining these factors. For instance, "transtelephonic rhythm strip pacemaker evaluation" is the beginning of the definition of a specific Common Procedure Terminology (CPT) code 93293 which is widely recognized as a covered benefit by commercial and government payers. In this instance, the reimbursement would go to the organization that owns the interrogation system communicating with the pacemaker and is making the assessment of the pacemaker performance.

An alternative method of compensation to reimbursement is reimbursement as an administrative expense. The most significant example of this at the present time for health plans and government health program falls under the rubric "disease management". Disease management encompasses a number of clinical services performed by an organization (typically a for-profit disease management company) that engages health plan and health program beneficiaries with communications that are telephonic, digital or mail-based. In return, the organization receives a payment that is typically determined by either a per capita payment rate (usually calculated on per member per month basis) or a percent of the savings incurred by the health plan that can be attributable to the intervention. The attributable savings are usually identified by determining the difference between observed hospitalization and emergency room visit rates and comparing these with a historical trend.

As used herein a compensation entity is any entity which pays other entities and a compensation trigger is any payment based on performance based metrics which enable better compensation for health professional for better patient health.

Touch Points or Inputs may be defined as: Personal URL/Code, PID/uniquely identifiable disc (CD, DVD, BD) or any other uniquely identifiable optical media, Web, mobile phone communications, or proximity communications such as Bluetooth.

In some embodiments, a transfer may be enabled using a computer readable medium, e.g. a CD or DVD disc. Each CD or DVD disc may be manufactured with a Postscribed ID^{™} or PID code. One example of a Postscribed ID is described in US patent 7,178,087 B2 to Bentvelsen et. al, that allows an optical disc to contain a machine readable value applied post manufacturing. In some embodiments, the value is applied to the optical disc via a high powered laser. The value can be pre-defined and can be unique to each and every optical disc.

This unique PID code can be embedded into the disc data using a number of methods. In some embodiments, specialized equipment housing a high intensity laser is employed in the disc manufacturing process. The PID can be written as a 16, 32 or 64 or any other length byte code which can then be read-back by application software contained on the disc for use within the client software application, or transferred by digital transmission or any other communications standard. In some embodiments, transfer purely electronic ID's are contemplated.

During the manufacturing process of the disc, it is possible to print a unique barcode, datamatrix code or human readable information on each disc label using variable data printing. This printed code can be associated to the PID code embedded within the data of each disc using a relational database. Although specific formats for data, particularly a PID, may be provided, it should be apparent that other data formats can be suitable. Also, recording methods other than printing may be employed to affix or associate the data (e.g., PID) with a physical object such as a disc. Other types of physical objects may be used such as a solid-state memory device (flash memory, memory stick, battery-backed up Random Access Memory (RAM), etc.

The PID code on the disc can be assigned to an individual, e.g. a patient, at a first entity, e.g. the patients primary care physician, and associated with that individual's records contained at that first entity. The PID code, which does not contain any medical information related to the individual, can be transferred to a second entity, e.g. a Participating Healthcare Program, as a program referral by digital transmission, or physical delivery of the disc, e.g. by the individual. The unique ID, code can be transferred via other methods that are derived from the PID. For example, a program on the disc or associated with the e-Backbone 1250 could print a unique code on paper that can reference the PID so that the user does not have to always take the disc with them everywhere they go. This could be, for example, in the shape of an ID card.

The unique disc PID code is then read-back at the second entity on a computer and transferred into the second entity system as a condition of referral during registration to the Participating Healthcare program. Here at the second entity, the PID code is associated with the individual's records relevant to their participation in said program. Upon the individual's successful completion of the Participating Healthcare Program, the second entity transfers the PID code, minus any personal information back to the first entity, together with the information marking that the individual has successfully completed the Healthcare program, measurement, etc.

While the above discussion relates to PID codes on a computer readable medium, it is also contemplated that any personal device may be used similarly to a PID disc.

Any electronic (passive or active) device capable of providing a unique identification number that can be combined with a user number for transmission to a node in Fig 1. may be, but is not limited to, devices including a smart card with an Radio Frequency device, cell phone, electronic ID, or any other device capable of transmitting and/or receiving wireless information). Further, it is contemplated that the device could be the measurement device itself by which a user number can be associated with the medical measurement.

The transmission could be through one or more of the following: Radio Frequencies, telephone frequencies, land lines, web enabled communications and/or blue tooth enabled communications.

A personal device having uniquely identifiable associated number, for example a cell phone which has an International Mobile Equipment Unique Identification Numbers (IMEIs), can be associated with a User number at the first entity. By way of this invention we anticipate a need and conceive of a software application that runs on a mobile device such as a cell phone that can combine uniquely identifiable information, for example the phone's IMEIs and a user ID#. The software could also run on medical device, personal medical device, remote medical device. This combined number can provide a unique non guessable ID for transfer by the Users phone/device wirelessly to each of the points described in Fig 1. Like a PID disc with Video content a "WEB" enabled phone can transmit generic medical information to the user. This information can be updated and transmitted and specific instructions can be forwarded to WEB and non-web enabled personal device. This personal device can provide user to user information through the authentication center by wireless transmission.

Data transfers may occur from a first entity, upon referral to the second entity's Healthcare program, from a second entity back to the first entity, upon admission of the individual to said program, and from the second entity back to the first entity and optionally one or more third parties, upon successful completion of said program.

The first entity or second entity activate the transfer of the PID code through the client software application contained on the disc relevant to the business rules applied around the participation within a medical program.

In some embodiments a digital transfer occurs in which the transfer of the unique PID code contained on the disc from a local computer or through an internet connected computer is activated within the client software application contained within the disc itself. However, the transfer of the PID code does not have to be initiated just by a client application on the disc. It is possible to obtain PID information off of a disc via a server side application that is initiated via a web connection.

Using an internet connected computer, an individual at the first entity can initiate a transfer or upload of the PID code and associated information at the time of patient referral to a second entity program. Here the PID code is read back by the first entity computer and transferred through the Electronic Backbone Infrastructure, wherein it can be made available to a Participating second entity group. In some embodiments input is authenticated before an action is taken. For example, several PID discs are delivered to a medical professional, with each disc having a known Medical/Healthcare program ID and physician's practice information. The discs provide multiple layers of intelligence upon authentication, with specific marketing or educational info, program information, or other information relevant to an authenticated user. This information may be configured to have secure access, or to enable viral circulation. In this embodiment, the e-Backbone 1250 provides both authentication and digital routing, connecting patients to specific content.

Another option for transferring the PID code can include a local transfer, wherein a disc is hand carried by the patient (after having received the disc at the first entity) to the second entity on condition of enrollment to the second entities' Healthcare program. The disc is loaded into a computer at the second entity, wherein the PID code is read-back locally from the disc and uploaded into the second entities computer system.

When a transfer occurs, a determining step enables the secure data transmission. In some embodiments, the Electronic backbone system confirms the transfer and receipt of PID transactions between first entity and second entity partners. Using a PID token authentication system, it is possible to confirm the receipt of a transferred PID code from either entity. Therefore, business rules can then define the administrative policies surrounding the providing of the medical benefit.

As used herein, the term "determining" and grammatical variants thereof is used in an extremely broad sense. The term "determining" encompasses a wide variety of actions and therefore "determining" can include calculating, computing, processing, deriving, investigating, looking up (e.g., looking up in a table, a database or another data structure), ascertaining and the like. Also, "determining" can include receiving (e.g., receiving information), accessing (e.g., accessing data in a memory) and the like. Also, "determining" can include resolving, selecting, choosing, establishing, and the like.

The term "determining" does not imply certainty or absolute precision, and therefore "determining" can include estimating, predicting, guessing and the like.

The term "determining" does not imply that mathematical processing must be performed, and does not imply that numerical methods must be used, and does not imply that an algorithm or process is used.

The term "determining" does not imply that any particular device must be used. For example, a computer need not necessarily perform the determining.

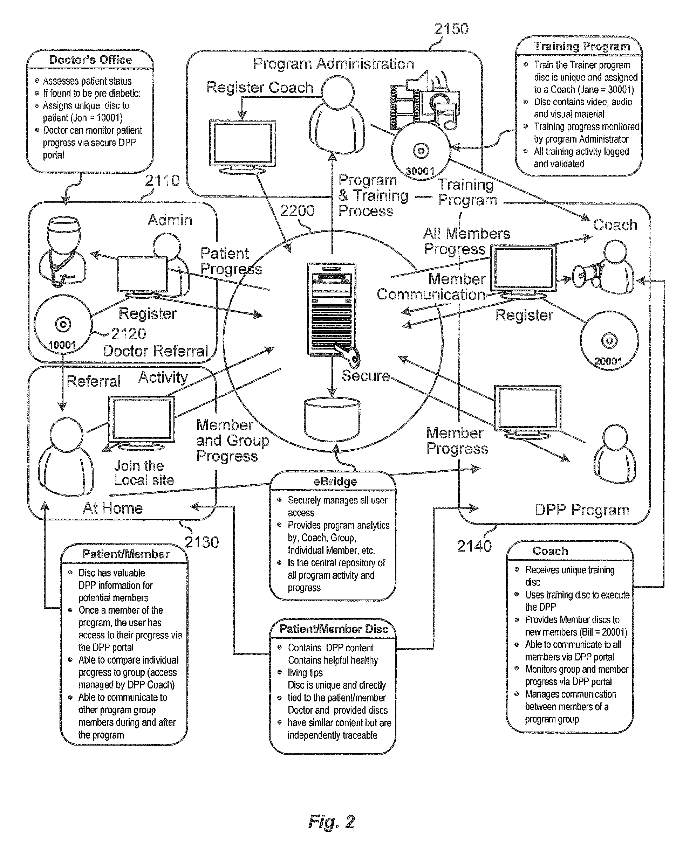

Figure 2 illustrates basic steps and objects/entities in a particular embodiment. In Figure 2, Digital Traffic Controller (DTC-2200) represents an entity that can initiate, manage, track, control or perform other functions, as desired, to facilitate operation of the system. In some embodiments it may be desirable for the DTC perform a minimal role. For example, the DTC may simply provide initial ID numbers in association with each particular patient. A DTC may also track transfer of content (e.g., medical record information in association with an ID number) among the various entities described, below. In one embodiment that uses an optical disc to transfer, at least in part, the content, the ID can be a Post-Scribed ID (PID) that is embedded on the optical media.

It should be apparent that other embodiments can use other forms of IDs and other types of distribution or media such as assignment or transfer of the IDs and other content by wired or wireless communication, magnetic or solid state media, voice communication, written printed media, etc. In alternative embodiments, the ID number can be associated with a device rather than a person as, for example, by using an IMEI number that is uniquely associated with a device. In other embodiments, different formats or types of a sufficiently secure ID, number, token, key or other secure identifier may be used.

As a first step the DTC transfers an "insignia" to an "initiator" entity such as a primary care provider that performs a diagnostic role for a patient. For example, the initiator can be a doctor, group of doctors, office, organization, etc. The insignia can include a digital mark that serializes a packet of information. In one embodiment the insignia does not reveal the source sender or content but allows the information to be directed to the prescribed recipient. For example, disc 2120 can be sent to office 2110 by mail from an undisclosed source.

The initiator associates a patient with the ID. Association can be by physical or electronic means. For example, a doctor can receive disc 2120 with a unique ID written onto the disc. The doctor can scan-in the ID on the disc by, for example, using a bar code that is labeled on the disc. The doctor can then store the scanned ID in association with the patient's medical records that are maintained confidentially in the doctor's offices or that are maintained and managed under the control of the doctor's office or by approved third parties or methods.

The digital insignia can be serialized to allow for greater protection from all other transmissions. For example, a batch of 500 discs going to a specific office can be in a sequence that is known to the DTC. In this manner if the specific office inquires about the status of patient records moving through the system the DTC can provide the information on a per-ID basis which the specific office can correlate to patients.

In a particular embodiment, once the initiating doctor (or the patient or another authorized person) puts the disc into the computer, the content is electronically and confidentially sent to a referral recipient. In a fully-automatic mode no typing is required since the transmission is directed automatically to, for example, doctor referral office 2130. The PID and any other information can all travel in the same associated transfer and the PID number is not guessable (e.g., not in numerical sequence with other numbers in other transfers). This number is only associated with a transmission not a person.

The DTC can detect and log the transfer from a doctor's office to referral agency 2100. In other embodiments the DTC does not need to detect the transfer. Such detection and logging can be used or not depending upon privacy issues, patient or doctor agreements, etc.

The referral agency sends content (e.g., medical records plus ID) to a healthcare provider such as DPP 2140 in Figure 2. Again, this could be tracked by the DTC system but does not have to be.

Program administration 2150 can provide access to health care services and make sure that each referral gets medical resources. Additionally, if program in insurance reimbursable the administrator could provide validation of successful completion for reimbursement to all parties in the digital network.

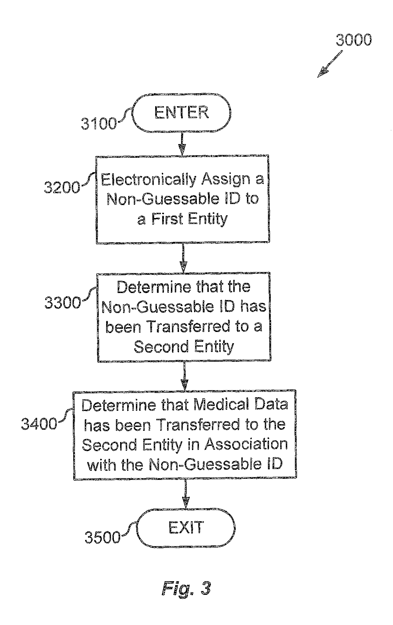

Fig. 3 illustrates basic steps in a flowchart of a routine suitable for implementing functionality according to an embodiment of the invention.

In Fig. 3, flowchart 3000 is entered at 3100 when it is desired to allow a customer or entity to transfer medical data. The steps illustrated in flowchart 3100 may be implemented by any suitable processing system such as a server computer coupled to a network. It should be apparent that steps or acts may be added to, removed from, or modified from those shown in Fig. 3. Fig. 3 is but one example of a simplified routine to achieve some of the functionality described herein.

Execution proceeds to 3200 where a Non-Guessable ID is assigned at a first entity. The first entity may then transfer the ID to a second entity. Such transfer is detected by the e-Backbone 1250 at 3300 and non-guessable ID is designated for information transfer in association with a medical record, however not containing the medical record itself. This information may include a referral recommendation.

At 3400 a medical data transfer in association with the ID is detected, and an authorized transaction is processed. This transaction may be confirming enrolment in a program at a medical benefits provider, updating status in that program, or authorizing a payment based on the users completion of a program.

The routine is exited at 3500.

Note that Figures 1, 2 and 3 are merely illustrations of possible systems suitable for use with embodiments of the invention. In other embodiments, the steps, entities, components or other items shown in Figures 1, 2 and 3 may be added, modified or omitted without departing from the scope of the claims.

Additional features or functionality can include providing for an automatic "level 1 code" for Medicare financial compensation or reimbursement upon referral to the provider. The referral can transfer without transfer of the medical records. The PID disc may only include an emblem that ties a unique serial number to a patient. In another embodiment the disc can provide medical information to the patient about the patient's disease. The system can automatically update the medical information once the disc is placed into the computer. The system can provide an electronic way to contact other patients or members in a defined group as allowed by the program administrator to allow for group positive reinforcement dynamics.

Various entities in the system can be allowed to inspect or receive data generated by or residing in the other parts of the system. For example, a patient or member could access levels of information for instruction or to maintain contact with their health care provider or referral recipient.

The PID information used by the patient in their computer or electronic device could be a signature for the gateway to send new updated confidential information to be provided by the health care provider or referral recipient. The PID or unique serialized code could be maintained by the patient permanently to access update information.

Other variations are possible. Any form of medical service or benefit can be provided such as a diagnosis, test, analysis, treatment, prescription, advice, subsequent referral, etc.

Although the description has been described with respect to particular embodiments thereof, these particular embodiments are merely illustrative, and not restrictive.

Any suitable programming language can be used to implement the routines of particular embodiments including C, C++, Java, assembly language, etc. Different programming techniques can be employed such as procedural or object oriented. The routines can execute on a single processing device or multiple processors. Although the steps, operations, or computations may be presented in a specific order, this order may be changed in different particular embodiments. In some particular embodiments, multiple steps shown as sequential in this specification can be performed at the same time.

Particular embodiments may be implemented in a computer-readable storage medium for use by or in connection with the instruction execution system, apparatus, system, or device. Particular embodiments can be implemented in the form of control logic in software or hardware or a combination of both. The control logic, when executed by one or more processors, may be operable to perform that which is described in particular embodiments.

Particular embodiments may be implemented by using a programmed general purpose digital computer, by using application specific integrated circuits, programmable logic devices, field programmable gate arrays, optical, chemical, biological, quantum or nanoengineered systems, components and mechanisms may be used. In general, the functions of particular embodiments can be achieved by any means as is known in the art. Distributed, networked systems, components, and/or circuits can be used. Communication, or transfer, of data may be wired, wireless, or by any other means.

It will also be appreciated that one or more of the elements depicted in the drawings/figures can also be implemented in a more separated or integrated manner, or even removed or rendered as inoperable in certain cases, as is useful in accordance with a particular application. It is also within the spirit and scope to implement a program or code that can be stored in a machine-readable medium to permit a computer to perform any of the methods described above.

As used in the description herein and throughout the claims that follow, "a", "an", and "the" includes plural references unless the context clearly dictates otherwise. Also, as used in the description herein and throughout the claims that follow, the meaning of "in" includes "in" and "on" unless the context clearly dictates otherwise.

The terms "an embodiment", "embodiment", "embodiments", "the embodiment", "the embodiments", "one or more embodiments", "some embodiments", "certain embodiments", "one embodiment", "another embodiment" and the like mean "one or more (but not all) embodiments of the disclosed invention(s)", unless expressly specified otherwise.

The term "variation" of an invention means an embodiment of the invention, unless expressly specified otherwise.

Web-Based Application shall mean an application that is accessible on the World Wide Web via any application capable of executing web based code. One example may be a web browser such as Microsoft's Internet Explorer. The application will be stored on a central server and accessed via other computers or devices.

Web-Based Form shall mean an electronic form used to enter information by an end user into a web-based application

A reference to "another embodiment" in describing an embodiment does not imply that the referenced embodiment is mutually exclusive with another embodiment (e.g., an embodiment described before the referenced embodiment), unless expressly specified otherwise.

The terms "including", "comprising" and variations thereof mean "including but not limited to", unless expressly specified otherwise.

The term "consisting of and variations thereof mean "including and limited to", unless expressly specified otherwise.

The term "plurality" means "two or more", unless expressly specified otherwise.

The term "herein" means "in this patent application, including anything which may be incorporated by reference", unless expressly specified otherwise.

The term "represent" and like terms are not exclusive, unless expressly specified otherwise. For example, the term "represents" do not mean "represents only", unless expressly specified otherwise. Similarly the term "portion" may mean "some, up to and including all".

The term "whereby" is used herein only to precede a clause or other set of words that express only the intended result, objective or consequence of something that is previously and explicitly recited. Thus, when the term "whereby" is used in a claim, the clause or other words that the term "whereby" modifies do not establish specific further limitations of the claim or otherwise restricts the meaning or scope of the claim.

The term "e.g." and like terms means "for example", and thus does not limit the term or phrase it explains.

It will be readily apparent to one of ordinary skill in the art that the various processes described herein may be implemented by, e.g., appropriately programmed general purpose computers and computing devices. Typically a processor (e.g., one or more microprocessors, one or more microcontrollers, one or more digital signal processors) will receive instructions (e.g., from a memory or like device), and execute those instructions, thereby performing one or more processes defined by those instructions.

A "processor" means one or more microprocessors, central processing units (CPUs), computing devices, microcontrollers, digital signal processors, or like devices or any combination thereof.

Thus a description of a process is likewise a description of an apparatus for performing the process. The apparatus can include, e.g., a processor and those input devices and output devices that are appropriate to perform the method.

Further, programs that implement such methods (as well as other types of data) may be stored and transmitted using a variety of media (e.g., computer readable media) in a number of manners. In some embodiments, hard-wired circuitry or custom hardware may be used in place of, or in combination with, some or all of the software instructions that can implement the processes of various embodiments. Thus, various combinations of hardware and software may be used instead of software only.

The term "computer-readable medium" refers to any medium that participates in providing data (e.g., instructions, data structures) which may be read by a computer, a processor or a like device. Such a medium may take many forms, including but not limited to, non-volatile media, volatile media, and transmission media. Non-volatile media include, for example, optical or magnetic discs and other persistent memory. Volatile media include dynamic random access memory (DRAM), which typically constitutes the main memory. Transmission media include coaxial cables, copper wire and fiber optics, including the wires that comprise a system bus coupled to the processor. Transmission media may include or convey acoustic waves, light waves and electromagnetic emissions, such as those generated during radio frequency (RF) and infrared (IR) data communications. Common forms of computer-readable media include, for example, a floppy disc, a flexible disc, hard disk, magnetic tape, any other magnetic medium, a CD-ROM, DVD, Blu-ray disc, any other optical medium, punch cards, paper tape, any other physical medium with patterns of holes, a RAM, a PROM, an EPROM, a FLASH-EEPROM, any other memory chip or cartridge, a carrier wave as described hereinafter, or any other medium from which a computer can read.

Various forms of computer readable media may be involved in carrying data (e.g. sequences of instructions) to a processor. For example, data may be (i) delivered from RAM to a processor; (ii) carried over a wireless transmission medium; (iii) formatted and/or transmitted according to numerous formats, standards or protocols, such as Ethernet (or IEEE 802.3), SAP, ATP, Bluetooth TM, and TCP/IP, TDMA, COMMA, and 3G; and/or (iv) encrypted to ensure privacy or prevent fraud in any of a variety of ways well known in the art.

Thus a description of a process is likewise a description of a computer-readable medium storing a program for performing the process. The computer-readable medium can store (in any appropriate format) those program elements which are appropriate to perform the method.

Just as the description of various steps in a process does not indicate that all the described steps are required, embodiments of an apparatus include a computer/computing device operable to perform some (but not necessarily all) of the described process.

Likewise, just as the description of various steps in a process does not indicate that all the described steps are required, embodiments of a computer-readable medium storing a program or data structure include a computer- readable medium storing a program that, when executed, can cause a processor to perform some (but not necessarily all) of the described process.

Where databases are described, it will be understood by one of ordinary skill in the art that (i) alternative database structures to those described may be readily employed, and (ii) other memory structures besides databases may be readily employed. Any illustrations or descriptions of any sample databases presented herein are illustrative arrangements for stored representations of information. Any number of other arrangements may be employed besides those suggested by, e.g., tables illustrated in drawings or elsewhere. Similarly, any illustrated entries of the databases represent exemplary information only; one of ordinary skill in the art will understand that the number and content of the entries can be different from those described herein. Further, despite any depiction of the databases as tables, other formats (including relational databases, object- based models and/or distributed databases) are well known and could be used to store and manipulate the data types described herein. Likewise, object methods or behaviors of a database can be used to implement various processes, such as the described herein. In addition, the databases may, in a known manner, be stored locally or remotely from any device(s) which access data in the database.

Various embodiments can be configured to work in a network environment including a computer that is in communication (e.g., via a communications network) with one or more devices. The computer may communicate with the devices directly or indirectly, via any wired or wireless medium (e.g. the Internet, LAN, WAN or Ethernet, Token Ring, a telephone line, a cable line, a radio channel, an optical communications line, commercial on-line service providers, bulletin board systems, a satellite communications link, a combination of any of the above). Each of the devices may themselves comprise computers or other computing devices, that are adapted to communicate with the computer. Any number and type of devices may be in communication with the computer.

In an embodiment, a server computer or centralized authority may not be necessary or desirable. For example, the present invention may, in an embodiment, be practiced on one or more devices without a central authority. In such an embodiment, any functions described herein as performed by the server computer or data described as stored on the server computer may instead be performed by or stored on one or more such devices.

In so far as the embodiments of the invention described above are implemented, at least in part, using software-controlled data processing apparatus, it will be appreciated that a computer program providing such software control and a transmission, storage or other medium by which such a computer program is provided are envisaged as aspects of the present invention.

Thus, white particular embodiments have been described herein, latitudes of modification, various changes, and substitutions are intended in the foregoing disclosure, and it will be appreciated that in some instances some features of particular embodiments will be employed without a corresponding use of other features without departing from the scope as set forth. Therefore, many modifications may be made to adapt a particular situation or material to the scope of the invention.

Although this disclosure describes a system that manipulates data, Applicant makes no claims to ownership rights in any of the manipulated data except for the ID number or other specific data that may be discussed and claimed herein.

Various respective aspects and features of the invention are defined in the appended claims. Combinations of features from the dependent claims may be combined with features of the independent claim as appropriate and not merely as explicitly set out in the claims.

## Claims

1. A system for dynamic health-related information transmission;
electronically assigning a unique ID to a first entity;
transferring the unique ID to a second entity
electronically determining that the unique ID has been transferred to a second entity; and
detecting a transmission of health-related data between the first and second entity.

2. The system of claim 1, wherein the first entity includes a record of the patient, wherein the unique ID is designated for association with the record.

3. The system of claim 1, wherein the health-related data includes a referral recommendation.

4. The system of claim 1 wherein a compensation transaction is communicated.

5. The system of claim 1 wherein the system associates the unique ID to a user.

6. The system of clam 1 wherein the unique ID is authenticated by a health-related benefits provider.

7. The system of clam 1 wherein the health-related data is not private health-related information.

8. A method for dynamic health-related information transmission, the method comprising the following performed by a processing system;
assigning a unique ID to a first entity;
determining that the unique ID has been transferred to a second entity; and detecting a transmission of health-related data transferred between the first and second entity in association with the unique ID.

9. An apparatus for dynamic health-related information transmission, the apparatus comprising
a processor;
a processor-readable device including instructions executable by the processor for:
assigning a unique ID to a first entity;
determining that the unique ID has been transferred to a second entity; and
detecting a transmission of health-related data transferred between the first and second entity in association with the unique ID.

10. A processor-readable device including instructions executable by a processor for dynamic health-related information transmission, the processor-readable device comprising one or more instructions for:
assigning a unique ID to a first entity;
determining that the unique ID has been transferred to a second entity; and
detecting a transmission of health-related data transferred between the first and second entity in association with the unique ID.
